# EUROPEAN PATENT APPLICATION

(11) **EP 2 719 288 A1**
(43) Date of publication of application: **16.04.2014**
(21) Application number: 13003866.4
(22) Date of filing: 02.08.2013
(51) Int. Cl.: A23L 1/30, A61K 45/00

(54) **COMPOSITION FOR THE TREATMENT OR PREVENTION OF DYSLIPIDAEMIAS**

(30) Priority: 12.10.2012 RO 201200725
(71) Applicant: Fulga, Ion Gigel, Bucuresti (RO); Vasile, Danut, Bucuresti (RO)
(72) Inventor: Fulga, Ion Gigel, Bucuresti (RO); Vasile, Danut, Bucuresti (RO)
(74) Representative: Tuluca, F. Doina

(57) **Abstract**

The invention is a composition consisting of a combination of a compound which decreases the synthesis of endogenous cholesterol with a compound that reduces cholesterol absorption, a compound that increases HDL and a compound that enhances cholesterol degradation, the ratio of the four compounds being 0.5...1: 0.5...1: 0.015: 1.5...2, expressed in parts by weight. Preferably, the composition according to the invention contains a combination of red rice yeast (or red rice extract) with niacin, sitostanol and taurine in oral pharmaceutical preparations to be used as food supplement or as medicine for the treatment or prevention of dyslipidaemias, for improving or preventing disturbances in the serum lipid profile.

## Description

The invention is a composition for pharmaceutical preparations to be used as food supplement or as medicine for the treatment or prevention of dyslipidaemias, for improving or preventing disturbances in the serum lipid profile.

Dyslipidaemias are today a major public health problem that is associated with accelerated atherosclerosis, which remarkably increases the risk of serious illness such as cardiovascular, cerebral, renal, etc.

The main body lipids circulating in the blood stream are the cholesterol, the phospholipids and the triglycerides. Some of these lipids are completely insoluble in water, i.e. hydrophobic; others have amphoteric molecules, with one hydrophilic and one hydrophobic end.

To be able to circulate in the plasma, it is imperative that these lipids assemble in groups (spherical particles) called lipoproteins. These lipoproteins consist of a hydrophobic core and a coating of amphoteric molecules having the hydrophobic end towards the inside of the lipoprotein and the hydrophilic end towards the outside, in contact with the aqueous medium represented by plasma. Inside, there are triglycerides and esterified cholesterol. The outer coating contains phospholipids, unesterified (free) cholesterol and some special proteins called apolipoproteins.

These lipoproteins were classified, according to their density, into very low density lipoproteins - VLDL, intermediate density lipoproteins - IDL, low density lipoproteins - LDL, and high density lipoproteins - HDL, plus chylomicrons. In maintaining the lipid homeostasis, lipoprotein undergoes some changes. VLDL, for example, turn into IDL, and IDL can be converted into LDL.

Elevated lipids, triglycerides and cholesterol are associated with an acceleration of the atherosclerotic process. Among the various forms of circulation of the cholesterol in the blood, the most dangerous in terms of acceleration of the atherosclerosis is without question the LDL cholesterol. Unlike the LDL cholesterol, HDL cholesterol appears to play, conversely, a role of protector against atherosclerosis. Therefore, a good hypolipidemic drug should not only lower the total cholesterol and LDL cholesterol, but, if possible, increase the HDL cholesterol, i.e. be not only hypolipidemic but a drug that improves the serum lipid profile.

The main drugs currently used to treat dyslipidaemias are statins (simvastatin (ZOCOR^{®}, VASILIP^{®}), atorvastatin (SORTIS^{®}), fluvastatin (LESCOL^{®}), lovastatin (MEVACOR^{®}), pravastatin (LIPOSTA^{®}), fibrates (clofibrate - first fibrate used in 1967, fenofibrate, gemfibrozil and bezafibrate) or other lipid-lowering agents such as bile acid sequestrants (such as cholestyramine and cholestipol) or nicotinic acid (Fulga I., Pharmacology, Ed. Medicala, 2004*,* Fulga I. Statins - Prototype Drugs for the Treatment of Dyslipidemia, MODERN MEDICINE, 2003 July 10 (7): 371-376)*.* These drugs known to treat dyslipidaemia can also produce significant adverse reactions.

Fenofibrate may accumulate in case of renal failure, sometimes causing dyspeptic disorders, rarely elevated transaminases, myositis phenomena, decreased libido, allergic reactions. Fenofibrate is contraindicated in hepatic impairment, renal failure, pregnancy and lactation, and the association with coumarin anticoagulants demands caution.

The main potential adverse effects of statins consist in raised liver transaminases and elevated creatine phosphokinase, sometimes accompanied by myositis phenomena. This makes necessary a control of liver enzymes and creatine phosphokinase levels every 2-3 months during the first 6 months of treatment, then twice a year. The combination with fibrates or nicotinic acid increases the risk of myopathy - caution is required, decreasing the dose of statins and more frequent monitoring of the creatine phosphokinase. Statins are incompatible with pregnancy.

Cholestyramine has an unfavourable benefit/risk ratio, and the peroral administration of high doses needed to treat hypercholesterolemias is unpleasant and can cause nausea, constipation, and flatulence. Cholestyramine sequesters in the intestine the acidic drugs, decreasing their availability for absorption.

Nicotinic acid is indicated in severe dyslipidaemias and it is considered a reserve drug, because the high doses needed frequently cause adverse reactions: gastrointestinal irritation, flush.

A very special interest is taken in the use of food supplements to improve the lipid profile based on well-known findings such as the favourable impact of the Mediterranean diet in this respect. Among these, there are many natural substances known or natural extracts that can influence the serum lipid profile.

One of the natural extracts that has a proven cholesterol-lowering effect is the red rice yeast or extract, which has the property of preventing the synthesis of cholesterol. Also, red rice yeast consistently reduces plasma lipids (Liu J, Zhang J, Shi Y, Grimsgaard S, Alraek T, Fønnebø V, Chinese red yeast rice (Monascus purpureus) for primary hyperlipidemia: a meta-analysis of randomized controlled trials, Chin Med, 2006 Nov. 23;1:4*).* The cholesterol-lowering effects of red rice (Heber D, Yip I, Ashley JM, Elashoff DA, Elashoff RM, Go VL, Cholesterol-lowering effects of a proprietary Chinese red-yeast-rice dietary supplement, Am J Clin Nutr, 1999 Feb;69(2):231-6*)* is due by fungi, namely the *Monascus species,* which develops on the surface of red rice grains. The most studied of these fungi is *Monascus purpureus* or *Monascus ruber.* Red rice yeast or red rice extract is produced by fermentation of the *Monascus species.* These products are obtained, after the fermentation of the non-agglutinated rice grains, by growing strains of *Monascus* in culture media, harvesting the culture and removing the environment. The residue containing the grown strains is dried and sterilized (GB 2046737 A) or extracted using ethanol (EP 10440096 B1) and constitutes the red rice extract. Some of the active ingredients of the red rice extract are monacolins (Huang HN, Hua YY, Bao GR, Xie LH, The quantification of monacolin K in some red yeast rice from Fujian province and the comparison of the other product.Chem Pharm Bull (Tokyo). 2006 May; 54(5):687-9*),* compounds similar to lovastatin and responsible for lowering serum triglyceride levels, as they are inhibitors of HMG-CoA reductase, thus preventing the synthesis of cholesterol (WO 2011068923 A par. 005).

Red rice yeast extract also contains other natural compounds such as: flavonoids, unsaturated fatty acids, and phytosterols. The combination of active compounds in the red rice extracts has inhibitory effects on the pro-oxidant status involved in atherogenesis.

Many food supplements that used as active ingredient only red rice extract are CHOLEST-BIO^{®} manufactured by Ropharma^{®}; Red Yeast Rice manufactured by Rexall^{®}, Solary^{®} or Smart Living^{®}; the medicinal product in the patent application US2012172425 (A1).

In recent years, formulas are known in which red rice extract is combined with other ingredients that contribute to the treatment of hypercholesterolemia. Thus, there are formulas in which the red rice extract is combined with various oils such as fish oil or polyunsaturated fatty acids such as omega-3 polyunsaturated acid (US 2012171285 A1; US 2012171311 A1; WO 2011068923 A1). The goal of these red rice extract combinations is to lower the total serum cholesterol, to lower the level of "bad" cholesterol (LDL) and/or to increase the level of "good" cholesterol (HDL), and to lower the triglycerides.

Another known combination of the red rice extract is the combination with *Cynara scolimus* leaf extract (FR 29367911 A1; EP 1967199 (A1)). This formula is used to combine the choleretic effect of the Cynara extract with the cholesterol synthesis inhibiting effect of the red rice extract, which means a stimulation of cholesterol excretion in the bile and a decrease in the total serum cholesterol levels.

A large number of other active ingredients with the ability to decrease serum cholesterol and triglyceride levels is known. The data available in the prior art show the following possible clinical uses of such ingredients:

It is very convincingly demonstrated that niacin increases the amount of HDL cholesterol *(*Ganji SH, Kamanna VS, Kashyap ML, Niacin and cholesterol: role in cardiovascular disease (review), J Nutr Biochem, 2003 Jun;14(6):298-305*. Review;* Luria MH, Effect of low-dose niacin on high-density lipoprotein cholesterol and total cholesterol/high-density lipoprotein cholesterol ratio, Arch Intern Med, 1988 Nov;148(11):2493-5*).* This substance also has a number of other beneficial effects such as the increase of the cholesterol efflux from cells, the normalization of vasomotricity, lowering of triglycerides, stopping the progress of the atherosclerosis process, all leading to a decrease in adverse clinical events in patients with coronary heart disease, this last fact being also demonstrated by convincing clinical studies (Alderman JD, Pasternak RC, Sacks FM, Smith HS, Monrad ES, Grossman W, Effect of a modified, well-tolerated niacin regimen on serum total cholesterol, high density lipoprotein cholesterol and the cholesterol to high density lipoprotein ratio, Am J Cardiol, 1989 Oct 1;64(12):725-9*;* Benjó AM, Maranhão RC, Coimbra SR, Andrade AC, Favarato D, Molina MS, Brandizzi LI, da Luz PL, Accumulation of chylomicrore remnants and impaired vascular reactivity occur in subjects with isolated low HDL cholesterol: effects of niacin treatment, Atherosclerosis. 2006 Jul;187(1):116-22. Epub 2006 Feb 3). It is true however that these effects are caused by high doses of niacin, pharmacological doses, not low doses such as those used in food supplements. Studies have been performed on the effects of the combination of niacin and simvastatin or lovastatin, studies which show that these combinations can increase HDL cholesterol levels (Ballantyne CM, Davidson MH, McKenney J, Keller LH, Bajorunas DR, Karas RH, Comparison of the safety and efficacy of a combination tablet of niacin extended release and simvastatin vs. simvastatin monotherapy in patients with increased non-HDL cholesterol (from the SEACOAST I study), Am J Cardiol, 2008 May 15;101(10):1428-36*;* Bays HE, Dujovne CA, McGovern ME, White TE, Kashyap ML, Hutcheson AG, Crouse JR; ADvicor Versus Other Cholesterol-Modulating Agents Trial Evaluation.Comparison of once-daily, niacin extended-release/ lovastatin with standard doses of atorvastatin and simvastatin (the ADvicor Versus Other Cholesterol-Modulating Agents Trial Evaluation [ADVOCATE]), Am J Cardiol., 2003 Mar 15; 91 (6):667-72*;* Yang J, Zhao SP, Li J, Wu ZH, Dong SZ, [Effect of niacin on HDL-induced cholesterol afflux and LXRalpha expression in adipocytes of hypercholesterolemic rabbits] Zhonghua Xin Xue Guan Bing Za Zhi. 2007 Aug; 35(8):745-9*, Chinese*)*.* Although it has been proven that niacin increases the amount of HDL cholesterol and lowers the serum triglyceride level, these effects are however significant at high doses of niacin, not the low doses such as those used in food supplements, for example.

It is known that sitosterol decreases cholesterol absorption, but only in amounts equivalent to those of cholesterol (Hassan AS, Rampone AJ, Intestinal absorption and lymphatic transport of cholesterol and beta-sitostanol in the rat, J Lipid Res, 1979 Jul;20(5):646-53*;* Aringer L, Eneroth P, Nordström L, Side chain hydroxylation of cholesterol, campesterol and beta-sitosterol in rat liver mitochondria, J Lipid Res. 1976 May;17(3):263-72*;* Aringer L, Eneroth P, Formation and metabolism in vitro of 5,6-epoxides of cholesterol and beta-sitosterol, J Lipid Res, 1974 Jul; 15(4): 389-98 *;* Aringer L, Eneroth P, Studies on the formation of C7-oxygenated cholesterol and beta-sitosterol metabolites in cell-free preparations of rat liver, J Lipid Res, 1973 Sep;14(5):563-7)*.* Sitostanol appears to decrease cholesterol absorption in the intestine better than sitosterol (Terry JG, McGill BL, Crouse JR 3rd, Evaluation of the use of beta-sitostanol as a nonabsorbable marker for quantifying cholesterol absorption, J Lipid Res, 1995 Oct; 36(10):2267-71*;* Borgström B, Quantitative aspects of the intestinal absorption and metabolism of cholesterol and beta-sitosterol in the rat, J Lipid Res, 1968 Jul; 9(4)473-81*;* Denke MA, Lack of efficacy of low-dose sitostanol therapy as an adjunct to a cholesterol-lowering diet in men with moderate hypercholesterolemia, Am J Clin Nutr, 1995 Feb;61(2):392-6*;* Field FJ, Mathur SN, beta-sitosterol: esterification by intestinal acylcoenzyme A: cholesterol acyltransferase (ACAT) and its effect on cholesterol esterification, J Lipid Res, 1983 Apr;24(4):409-17*;* Gerson T, Shorland FB, Dunckley GG, The effect of beta-sitosterol on the metabolism of cholesterol and lipids in rats on a diet containing coconut oil, Biochem J, 1965 Aug; 96(2):399-403*;* Gylling H, Radhakrishnan R, Miettinen TA, Reduction of serum cholesterol in postmenopausal women with previous myocardial infarction and cholesterol malabsorption induced by dietary sitostanol ester margarine: women and dietary sitostanol, Circulation, 1997 Dec 16;96(12):4226-31*;* Nair PP, Turjman N, Kessie G, Calkins B, Goodman GT, Davidovitz H, Nimmagadda G, Diet, nutrition intake, and metabolism in populations at high and low risk for colon cancer. Dietary cholesterol, beta-sitosterol, and stigmasterol, Am J Clin Nutr, 1984 Oct;40(4 Suppl):927-30*;* Jones PJ, Ntanios FY, Raeini-Sarjaz M, Vanstone CA, Cholesterol-lowering efficacy of a sitostanol-containing phytosterol mixture with a prudent diet in hyperlipidemic men, Am J Clin Nutr, 1999 Jun; 69(6):1144-50)*.*

Moreover, a limitation for obtaining the therapeutic effects of is also the fact that the sitosterol reduces the absorption of cholesterol, but only in amounts equivalent to those of cholesterol. Unlike sitostanol, sitosterol appears to have influence only as food product.

Carnitine may increase HDL cholesterol and decrease triglycerides, but this was demonstrated only in haemodialysis patients and only at very high doses (Vacha GM, Giorcelli G, Siliprandi N, Corsi M, Favorable effects of L-carnitine treatment on hypertriglyceridemia in hemodialysis patients: decisive role of low levels of high-density lipoprotein-cholesterol, Am J Clin Nutr, 1983 Oct;38(4):532-40*).*

It is known that taurine reduces the changes induced by atherosclerosis, enhances the degradation of cholesterol and may increase the HDL cholesterol concentration (Mochizuki H, Oda H, Yokogoshi H, Increasing effect of dietary taurine on the serum HDL-cholesterol concentration in rats, Biosci Biotechnol Biochem, 1998 Mar;62(3):578-9*).* Unfortunately, all these results were obtained only by non clinical studies, and there are no results obtained using taurine in clinical trials. (Balkan J, Oztezcan S, Hatipoglu A, Cevikbas U, Aykac-Toker G, Uysal M, Effect of a taurine treatment on the regression of existing atherosclerotic lesions in rabbits fed on a high-cholesterol diet, Biosci Biotechnol Biochem, 2004 May; 68(5):1035-9*; Balkan J, Kanba* 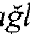 *li O, Hatipo* 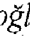 *lu A, Kücük M, Cevikba* 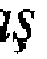 *U, Aykaç-Toker G, Uysal M,* Improving effect of dietary taurine supplementation on the oxidative stress and lipid levels in the plasma, liver and aorta of rabbits fed on a high-cholesterol diet, Biosci Biotechnol Biochem, 2002 Aug;66(8):1755-8*;* Balkan J, Oztezcan S, Aykaç-Toker G, Uysal M, Effects of added dietary taurine on erythrocyte lipids and oxidative stress in rabbits fed a high cholesterol diet, Biosci Biotechnol Biochem, 2002 Dec; 66(12):2701-5)*.*

Quercetin may decrease LDL cholesterol levels and slow down the progress of atherosclerosis. The studies are however very few and the reproducibility of the data is not demonstrated. (Arai Y, Watanabe S, Kimira M, Shimoi K, Mochizuki R, Kinae N, Dietary intakes of flavonols, flavones and isoflavones by Japanese women and the inverse correlation between quercetin intake and plasma LDL cholesterol concentration, J Nutr, 2000 Sep;130(9):2243-50*;* Chien JT, Hsu DJ, Chen BH, Kinetic model for studying the effect of quercetin on cholesterol oxidation during heatin, J Agric Food Chem, 2006 Feb 22;54(4):1486-92*;* Enkhmaa B, Shiwaku K, Katsube T, Kitajima K, Anuurad E, Yamasaki M, Yamane Y, Mulberry (Morus alba L.) leaves and their major flavonol quercetin 3-(6-malonylglucoside) attenuate atherosclerotic lesion development in LDL receptor-deficient mice, J Nutr, 2005 Apr;135(4): 729-34 Field FJ, Born E, Mathur SN, Effect of micellar beta-sitosterol on cholesterol metabolism in CaCo-2 cells, J Lipid Res, 1997 Feb;38(2):348-60*;)* Kamada C, da Silva EL, Ohnishi-Kameyama M, Moon JH, Terao J, Attenuation of lipid peroxidation and hyperlipidaemia by quercetin glucoside in the aorta of high cholesterol-fed rabbit. Free Radic Res. 2005 Feb;39(2):185-94*).*

Resveratrol may decrease LDL and increase HDL (Dávalos A, Fernández-Hernando C, Cerrato F, Martinez-Botas J, Gómez-Coronado D, Gómez-Cordovés C, Lasunción MA, Red grape juice polyphenols alter cholesterol homeostasis and increase LDL-receptor activity in human cells in vitro, J Nutr, 2006 Jul;136(7):1766-73*. Review;* Do GM, Kwon EY, Kim HJ, Jeon SM, Ha TY, Park T, Choi MS, Long-term effects of resveratrol supplementation on suppression of atherogenic lesion formation and cholesterol synthesis in apo E-deficient mice, Biochem Biophys Res Commun, 2008 Sep 12;374(1):55-9. Epub 2008 Jul 9*),* but these effects have only been proven in certain types of genetically modified mice and only by non clinical studies.

A further disadvantage of the use of the substances mentioned above is that they all have effects only at high doses, higher than those used as necessary in food supplements.

The problem solved by this invention is a new combination of certain categories of active ingredients, while ensuring synergetic effects in the treatment of hypercholesterolemia and hyperlipidaemia.

The composition for the prevention and treatment of dyslipidaemia, according to the invention, consists in a combination of a compound which lowers the synthesis of endogenous cholesterol, a compound that reduces cholesterol absorption, a compound which increases HDL and a compound that enhances cholesterol degradation, the ratio being of 0.5...1: 0.5...1: 0.015: 1.5...2, expressed in parts by weight.

The composition, according to the invention, consists, in the preferred form, in a combination of red rice extract which lowers cholesterol synthesis and significantly reduces serum lipids with a compound that reduces cholesterol absorption, a compound which contributes to the degradation of cholesterol and a compound which increases the HDL cholesterol and/or lowers the LDL cholesterol in a combination ratio of 1:1:0.015:1.5, expressed in parts by weight.

In this composition, according to the invention, in the preferred form, the compound having an effect of reducing cholesterol absorption is chosen from sitosterol and sitostanol, the compound that contributes to the degradation of cholesterol is taurine, and the compound that has the effect of increasing the HDL cholesterol is either niacin, or carnitine or resveratrol.

The preferred composition according to the invention consists of a combination of 28.45% red rice extract (or red rice yeast) containing min. 1% monacolin K, with 28.45% sitostanol containing min. 70% beta-sitosterol, with 0.428% niacin and 42.67% taurine.

The advantages of the composition according to the invention are the following:
- significantly improves the serum lipid profile by lowering the total lipid concentration, the total cholesterol and the LDL cholesterol and increasing the HDL cholesterol;
- is made of natural ingredients, normally used as food supplements and so, by definition, very well tolerated.

Examples are given below to illustrate the invention in conjunction with the figures representing:
- Figure 1: impact on the lipid profile in rats of the cholesterol rich diet; column height represents the increase in serum lipid levels (mg/dL) at 2 weeks and 4 weeks, respectively, after the start of the experiment compared to baseline;
- Figure 2: impact on the lipid profile by two combinations T tested in rats fed on cholesterol rich diet. Column height represents the decrease in serum lipid levels (mg/dL) at 2 weeks after the start of the experiment compared to baseline.
- Figure 3: impact on the HDL cholesterol concentration by the two combinations T tested by comparison with untreated animals in rats fed on cholesterol rich diet. Column height represents the increase in the serum HDL cholesterol levels (mg/dL) at 2 weeks after the start of the experiment compared to baseline.

In addressing the goal set, namely that of obtaining a combination of active ingredients with therapeutic effects in the treatment of hypercholesterolemia, we chose the ingredients which have, by combination, the probability of a synergetic effect in this treatment and especially those that are found in nature. The known cholesterol-lowering and lipid-lowering properties of the chosen ingredients were studied.

The study showed that from the point of view of their known therapeutic effects, the studied compounds can be grouped into four categories:
A. *Compounds that increase the HDL cholesterol,* of which the following are retained:
   niacin, carnitine and resveratrol.
B. *Compounds that reduce cholesterol absorption,* of which the following are retained: sitoserol, sitostanol and quercetin.
C. *Compounds that lower the synthesis of cholesterol,* of which red rice extract or yeast is retained.
D. *Compounds that enhance cholesterol degradation,* of which taurine is retained.

As noted above, niacin is a well known compound and there are studies about it increasing the HDL cholesterol level. In a clinical study conducted in 19 men with HDL deficiency (HDL cholesterol < 5th percentile) aged 55 +/-10 years, the average change in HDL cholesterol caused by atorvastatin, fenofibrate and niacin was -6% (p = NS), 6% (p = NS), and + 22% (p <0.05) respectively (Alrasadi K, Awan Z, Alwaili K, Ruel I, Hafiane A, Krimbou L, Genest J, Comparison of treatment of severe high-density lipoprotein cholesterol deficiency in men with daily atorvastatin (20 mg) versus fenofibrate (200 mg) versus extended-release niacin (2 g), Am J Cardiol, 2008 Nov 15;102(10):1341-7. Epub 2008 Sep 11).

A further study tested the effect of decreasing phosphate and increasing HDL cholesterol produced by Niaspan (extended release nicotinic acid) in 20 patients on chronic dialysis, treated for 12 weeks. There was a significant increase in HDL cholesterol from 40 +/-3.2 to 59 +/-5.5 mg/dL (34%) after the administration of Niaspan (P = 0.0005). The study concluded that Niaspan is effective in increasing HDL cholesterol (Müller D, Mehling H, Otto B, Bergmann-Lips R, Luft F, Jordan J, Kettritz R, Niacin lowers serum phosphate and increases HDL cholesterol in dialysis patients, Clin J Am Soc Nephrol, 2007 Nov;2(6):1249-54, Epub 2007 Oct 3).

A study aimed to investigate the effect of niacin on HDL-induced cholesterol efflux in adipocytes from hypercholesterolemic rabbits. After 6 weeks of treatment, niacin significantly increased the cholesterol efflux from adipocytes (Yang J, Zhao SP, Li J, Wu ZH, Dong SZ, [Effect of niacin on HDL-induced cholesterol efflux and LXRalpha expression in adipocytes of hypercholesterolemic rabbits] Zhonghua Xin Xue Guan Bing Za Zhi, 2007 Aug;35(8):745-9*, Chinese).*

In a clinical trial, thirteen men with HDL < 1.04 mmol/L and no other risk factors for coronary artery disease were studied and 11 normal subjects used as a control group with HDL > 1.04 mmol/L. Flow-mediated dilation (FMD) decreased in those with low HDL (7.4 +/-4.1 versus 12.8 +/-4.6%, p < 0.001), while nitrite-mediated dilation was similar in both groups. After 3 months of treatment, plasma lipid and chylomicron kinetics were not altered by the treatment with niacin, but FMD increased to normal levels (from 5.44 +/-1.89 to 11.13 +/- 3.4%, p < 0.01) *(*Benjó AM, Maranhão RC, Coimbra SR, Andrade AC, Favarato D, Molina MS, Brandizzi LI, da Luz PL, Accumulation of chylomicron remnants and impaired vascular reactivity occur in subjects with isolated low HDL cholesterol: effects of niacin treatment. Atherosclerosis, 2006 Jul;187(1):116-22, Epub 2006 Feb 3*).*

In a literature study, the authors show that niacin is the most potent available drug used to lower plasma triglycerides and increase HDL cholesterol levels (Chrysant SG, Ibrahim M. Niacin-ER/statin combination for the treatment of dyslipidaemia: focus on low high-density lipoprotein cholesterol. J Clin Hypertens (Greenwich). 2006 Jul;8(7):493-9*; quiz 500-1. Review).*

Zhao XQ (Zhao XQ, Morse JS, Dowdy AA, Heise N, DeAngelis D, Frohlich J, Chait A, Albers JJ, Brown BG, Safety and tolerability of simvastatin plus niacin in patients with coronary artery disease and low high-density lipoprotein cholesterol (The HDL Atherosclerosis Treatment Study), Am J Cardiol, 2004 Feb 1;93(3):307-12*)* shows that the study "The high-density lipoprotein (HDL)-Atherosclerosis Treatment Study" demonstrated that simvastatin plus niacin (average daily dose of 13 mg and 2.4 g, respectively) stops the progress of atherosclerosis and reduces by 60% the major clinical events in patients with coronary artery disease who have low levels of HDL, compared with placebo, over 3 years of treatment.

Sitostanol decreases the absorption of cholesterol in the intestine. In a clinical study it was proven that beta-sitostanol inhibits cholesterol absorption (Normén L, Dutta P, Lia A, Andersson H, Soy sterol esters and beta-sitostanol ester as inhibitors of cholesterol absorption in human small bowel.Am J Clin Nutr. 2000 Apr;71(4):908-13*).* Thus, powder sitostanol (1 g) reduced the absorption of cholesterol only by 11.3 +/7.4% (P = 0.2). In contrast, sitostanol in lecithin micelles reduced cholesterol absorption by 36.7 +/- 4.2% (P = 0.003) at a dose of 700 mg, and by 34.4 +/- 5.8% (P = 0.01) at a dose of 300 mg (Ostlund RE Jr, Spilburg CA, Stenson WF, Sitostanol administered in lecithin micelles potently reduces cholesterol absorption in humans, Am J Clin Nutr, 1999 Nov;70(5):826-31*).*

The use of dietary sitostanol ester margarine showed that this ester normalizes LDL cholesterol in approximately one third of women with a history of myocardial infarction, especially in those with a high rate of cholesterol absorption and low cholesterol synthesis *(*Gylling H, Radhakrishnan R, Miettinen TA, Reduction of serum cholesterol in postmenopausal women with previous myocardial infarction and cholesterol malabsorption induced by dietary sitostanol ester margarine: women and dietary sitostanol. Circulation, 1997 Dec 16;96(12):4226-31*).* Also, the use of dietary sitostanol margarine normalizes blood lipids in patients with mild hypercholesterolemia reducing in particular the LDL cholesterol (Miettinen TA, Puska P, Gylling H, Vanhanen H, Vartiainen E, Reduction of serum cholesterol with sitostanol-ester margarine in a mildly hypercholesterolemic population, N Engl J Med, 1995 Nov 16;333(20):1308-12*).*

The cholesterol-lowering effects of red rice were demonstrated (Heber D, Yip I, Ashley JM, Elashoff DA, Elashoff RM, Go VL, Cholesterol-lowering effects of a proprietary Chinese red-yeast-rice dietary supplement, Am J Clin Nutr, 1999 Feb; 69(2):231-6*).* Red rice also significantly reduces plasma lipids. Red rice yeast reduces the adverse cardiac events in a manner similar to statins. Red rice yeast significantly reduces the total cholesterol, the LDL cholesterol and the total triglyceride concentration compared to placebo *(*Ong HT, Cheah JS, Statin alternatives or just placebo: an objective review of omega-3, red yeast rice and garlic in cardiovascular therapeutics, Chin Med J (Engl), 2008 Aug 20;121 (16):1588-94*. Review).*

In a study in rabbits, it was found that the extent of atherosclerotic lesions in the aorta was lower in the group treated with taurine than in the control group. Taurine appears to reduce the changes induced by atherosclerosis. These studies prove that taurine may accelerate the regression of atherosclerotic lesions induced by cholesterol (Balkan J, Oztezcan S, Hatipoglu A, Cevikbas U, Aykac-Toker G, Uysal M, Effect of a taurine treatment on the regression of existing atherosclerotic lesions in rabbits fed on a high-cholesterol diet, Biosci Biotechnol Biochem. 2004 May;68(5):1035-9). A study in rabbits shows that taurine improved the oxidative stress and the accumulation of cholesterol in the aorta of rabbits fed on a cholesterol-rich diet, and that this effect could be related to its antioxidant potential (Balkan J, Kanbagli O, Hatipoglu A, Kücük M, Cevikba 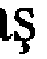 U, Aykaç-Toker G, Uysal M, Improving effect of dietary taurine supplementation on the oxidative stress and lipid levels in the plasma, liver and aorta of rabbits fed on a high-cholesterol diet, Biosci Biotechnol Biochem, 2002 Aug;66(8):1755-8).

A study conducted in diabetic rats shows that one of the mechanisms by which taurine has a hypolipidemic effect could be the enhancement of cholesterol degradation (Mochizuki H, Takido J, Oda H, Yokogoshi H. Improving effect of dietary taurine on marked hypercholesterolemia induced by a high-cholesterol diet in streptozotocin-induced diabetic rats, Biosci Biotechnol Biochem, 1999 Nov;63(11):1984-7*).*

A study in rats fed on a cholesterol-rich diet reaches the same conclusions as the previous study: one of the mechanisms by which taurine has hypolipidemic effect could be the enhancement of cholesterol degradation (Yokogoshi H, Mochizuki H, Nanami K, Hida Y, Miyachi F, Oda H, Dietary taurine enhances cholesterol degradation and reduces serum and liver cholesterol concentrations in rats fed a high-cholesterol diet, J Nutr, 1999 Sep;129(9):1705-12*).* A study in rats shows that taurine increased the serum HDL cholesterol concentration in a dose-dependent manner, without affecting in any way the total cholesterol *(*Mochizuki H, Oda H, Yokogoshi H.Increasing effect of dietary taurine on the serum HDL cholesterol concentration in rats, Biosci Biotechnol Biochem, 1998 Mar;62(3):578-9*).*

Knowing these data from the prior art, we have chosen the desired compounds from the categories of compounds that increase the HDL cholesterol (category A), compounds that lower the synthesis of endogenous cholesterol (category C), compounds that reduce cholesterol absorption (category B) and compounds that enhance cholesterol degradation (category D), in order to achieve a combination of representatives of these categories in a pharmaceutical product or food supplement.

The studies conducted by the authors of this invention regarding such combinations have shown that the effects of the combination are stronger than the effects of the individual compounds, which means that, in fact, a synergy between their actions is obtained in respect of the increase of the HDL cholesterol level, the reduction of cholesterol absorption, the lowering of cholesterol synthesis and the enhancement of cholesterol degradation.

Although there is no study in the literature evaluating such combinations or associations, the combination according to the invention has been studied and it was found that the mechanism of action of the combination appears to be a complex one, intervening in almost all metabolic processes of the body lipids.

Having regard to the 4 categories of compounds (A, B, C and D) whose therapeutic effects in the treatment of hypercholesterolemia were proven and known in the prior art, for achieving this invention some possible combinations of these compounds have been studied.

The possibilities of combination of the compounds in the categories A, B, C and D are presented below in Table 1.

**Table 1 - Studied combinations of compounds in the four categories:**

| **No.** | **Category** | **A** | **B** | **C** | **D** |
|---|---|---|---|---|---|
| | | **Niacin** | **Sitosterol** | **Red rice yeast** (or red rice extract) | **Taurine** |
| | | **Carnitine** | **Sitostanol** | | |
| | | **Resveratrol** | **Quercetin** | | |
| | **Combination** | | **Resveratrol** | | |
| 1. | **Combination T** | Niacin | Sitosterol | Red rice yeast | Taurine |
| 2. | **Combination** S | Niacin | Sitostanol | Red rice yeast | Taurine |
| 3. | **Combination** U | Niacin | Quercetin | Red rice yeast | Taurine |
| 4. | **Combination** V | Niacin | Resveratrol | Red rice yeast | Taurine |
| 5 | **Combination** Y | Carnitine | Sitosterol | Red rice yeast | Taurine |
| 6. | **Combination** AA | Carnitine | Sitostanol | Red rice yeast | Taurine |
| 7 | **Combination** BB | Carnitine | Quercetin | Red rice yeast | Taurine |
| 8 | **Combination** CC | Carnitine | Resveratrol | Red rice yeast | Taurine |
| 9 | **Combination** FF | Resveratrol | Sitosterol | Red rice yeast | Taurine |
| 10 | **Combination** GG | Resveratrol | Sitostanol | Red rice yeast | Taurine |
| 11 | **Combination** HH | Resveratrol | Quercetin | Red rice yeast | Taurine |
| 12 | **Combination** JJ | Resveratrol | Resveratrol | Red rice yeast | Taurine |

In order to evaluate which of these combinations has indeed a favourable effect on the serum lipid profile, a number of experimental studies were conducted in rats, with the clear goal of obtaining a synergetic effect of the active ingredients. To this end, the most effective combination in a food supplement was that of a compound which increases HDL cholesterol (category A) with a compound that lowers the synthesis of endogenous cholesterol (category C), a compound that reduces cholesterol absorption (category B) and a compound that enhances cholesterol degradation (category D), i.e. a combination of compounds in all four categories.

The findings were surprising, as the combinations of compounds affected most of the functional links of the lipid system in the body with appropriate therapeutic effect, much more obvious than that of the individual compounds, even though each compound was used in subtherapeutic doses. This hypothesis was demonstrated experimentally, as the literature currently does not provide any information in this regard on such combinations.

A first study was conducted on male Wistar rats with initial weight of approximately 180 grams. The rats were housed individually in Plexiglas cages whose floor was covered with absorbent sawdust and were brought to the laboratory five days prior to the experiment for acclimatization. Throughout the experiment, rats had free access to food and water. The cages were placed in the same room throughout the experiment, and a relatively constant temperature (∼ 22°C) was maintained.

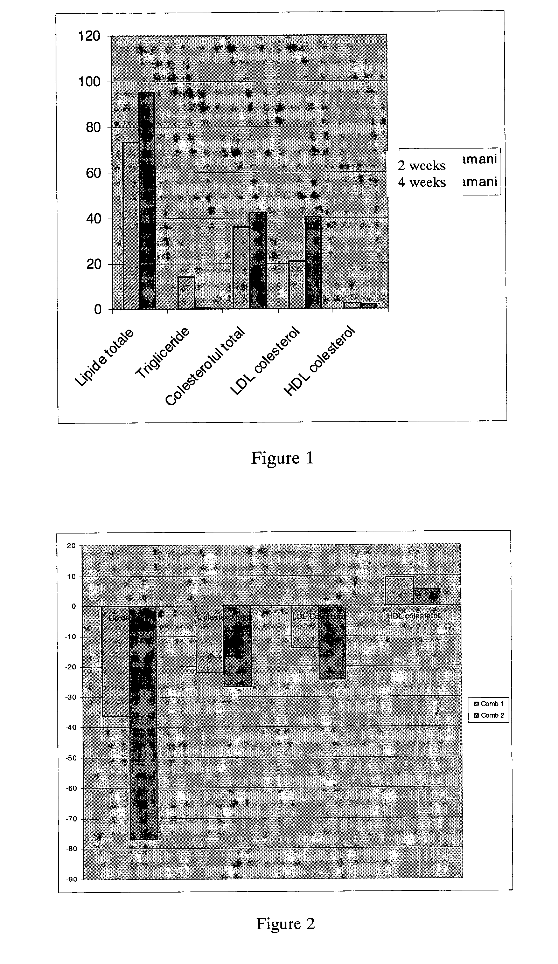

To induce hypercholesterolemia, the rats were fed cholesterol-enriched food, fats accounting for 35% - prepared by a firm specializing in diets for laboratory animals. The cholesterol-enriched food was administered for 4 weeks. Biochemical tests were performed prior to administration of the cholesterol-enriched food, at 2 and 4 weeks after its administration. The biochemical test results are shown in Figure 1, with the total cholesterol, LDL-cholesterol, HDL-cholesterol, triglycerides, total lipids on the y-coordinate and the lipid concentrations and 2 weeks and 4 weeks respectively after the start of the experiment compared to baseline on the x-coordinate. Column height represents the increase in the serum lipid levels (mg/dL).

The results of the research presented in Figure 1 show that the cholesterol rich diet worsens the serum lipid profile. Under the effect of this type of diet the concentration of total lipids, total cholesterol and LDL cholesterol increases. HDL cholesterol increases insignificantly and an increase in triglycerides is noted after 2 weeks of cholesterol rich diet, but the triglyceride concentration returns to normal after 4 weeks of such food. In these circumstances, it was decided that the experimental model was suitable for studying the impact on total lipid, total cholesterol, LDL cholesterol and HDL cholesterol levels, but not on the serum triglyceride level.

All the combinations in Table 1 were then administered by batches of rats fed on cholesterol rich diet. The evaluation of the combinations in Table 1 and of the individual compounds showed that the best combination is the combination T, which contains niacin, sitosterol, red rice yeast (or red rice extract) and taurine.

An experimental study monitored how the combination T affects the lipid profile in rats fed with cholesterol-enriched food. The rats were housed individually in Plexiglas cages whose floor was covered with absorbent sawdust and were brought to the laboratory five days prior to the experiment for acclimatization. Throughout the experiment, rats had free access to food and water. The cages were placed in the same room throughout the experiment, and a relatively constant temperature (∼ 22°C) was maintained.

Two treatment options were studied for the combination T, noted Combination 1, which contains red rice yeast extract 1000 mg/day + sitosterol 300 mg/day + niacin 8 mg/day + taurine 300 mg/day, and Combination 2, which contains red rice yeast extract 300 mg/day + sitosterol 1,125 mg/day + niacin 9 mg/day + taurine 150 mg/day. The administration of the treatment started on the same day with that of the cholesterol-enriched food. Biochemical tests were performed prior to administration of the cholesterol-enriched food and 2 weeks after its administration. The biochemical tests are: total cholesterol, LDL cholesterol, HDL cholesterol, total lipids.

The results of the research are presented in Figure 2 and the graph in this figure shows the impact on the lipid profile of the two combinations tested in rats fed with cholesterol-enriched food. Column height represents the decrease in the serum lipid levels (mg/dL) at 2 weeks after the start of the experiment compared to baseline.

As one can see, despite the administration of cholesterol rich food, serum levels of total lipids, total cholesterol and LDL cholesterol do not increase the same as in the graph in Figure 1, on the contrary, they all decrease. In contrast, the serum level of HDL cholesterol, which protects against atherosclerosis, increases.

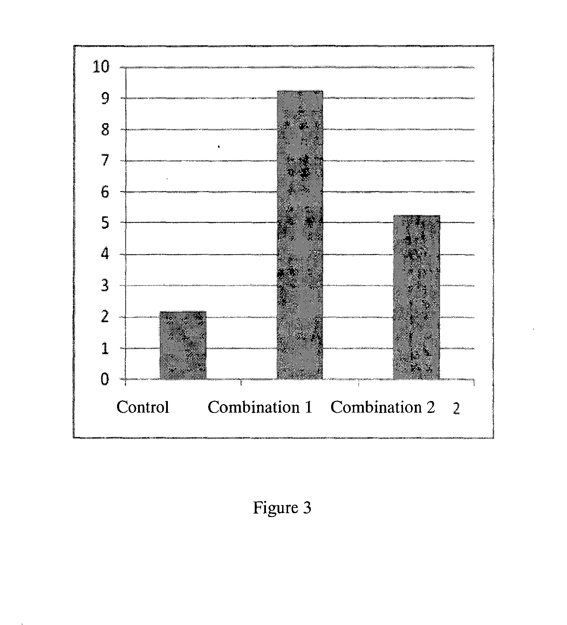

A very special problem in terms of clinical importance is the increase in the proportion of HDL cholesterol, since this type of cholesterol has a protective effect against strokes and heart diseases. In general, with classical lipid-lowering drugs such an increase in the proportion of HDL cholesterol is observed, but generally it is considered a relative increase rather due to the reduction of the weight of LDL cholesterol and not to an absolute increase in the HDL cholesterol level. In order to determine what happens in fact, for the combination making the subject of this invention the absolute values of HDL cholesterol in rats fed a cholesterol rich diet were compared to those in rats which received, in addition to the cholesterol rich diet, a treatment with the two studied combinations T, namely Combination 1 and Combination 2. The results are shown in Figure 3. Column height represents the increase in the serum HDL cholesterol level (mg/dL) at 2 weeks after the start of the experiment compared to baseline.

As one can see in the graph in Figure 3, in the animals fed with cholesterol-enriched food the HDL cholesterol increased by 2.16 mg/dL, while the cholesterol-enriched food administered together with the combination T resulted in increases in the HDL cholesterol level by 9.25 mg/dL for Combination 1 and 5.26 mg/dL for Combination 2, suggesting an absolute increase in the HDL cholesterol level and not a relative increase, as was the case for conventional lipid-lowering drugs.

From the data presented above, it is obvious that this Combination T clearly improves the serum lipid profile.

Also, all of the above shows that the combination of niacin, sitostanol, red rice yeast and taurine can be used in pharmaceutical preparations as a food supplement or as a medicine for the treatment or prevention of dyslipidaemia, in order to improve or prevent disturbances in the serum lipid profile.

Once the results of the experiments were obtained, we determined the compositions required for the manufacture of pharmaceutical products or food supplements and their dosage forms. Given the possible impact of the combination according to the invention on the intestinal absorption of cholesterol, dosage forms administered by mouth are preferable, whether these are solid or liquid.

The composition according to this invention is intended for oral administration in the form of capsules, tablets, aqueous suspension or oil-in-water emulsion.

All the active ingredients of the composition according to the invention are available on the market (Xiamen Forever Green Source Biochem Tech. Co. Ltd.^{®}, China, DSM Corporate^{®} and others). The composition according to the invention is prepared by mixing uniformly and thoroughly the four active ingredients in the categories A, B, C and D and then conditioning the mixture using pharmaceutically acceptable composition excipients, known per se, to obtain each desired dosage form.

Thus, the composition according to the invention can be formulated as tablets using diluting agents such as lactose or microcrystalline cellulose, lubricating agents such as magnesium stearate or talc, granulating agents such as hydroxypropylmethyl cellulose and pregelatinised maize starch, disintegrating agents such as corn starch or other agents known per se for formulating tablets.

Encapsulation in hard-shelled capsules is done by using a diluent such as lactose, and a glidant. Soft capsules used for formulating the composition according to the invention are those made of gelatine and glycerine.

A suspension of the mixture of active ingredients can be prepared by suspending it in water using the suspension or dispersion additives known per se, such as cellulose derivatives or sorbitol syrup, and preservatives such as methyl or propyl p-hydroxybenzoates.

The composition according to the invention is used in pharmaceutical oral preparations as food supplement or as medicine for the treatment or prevention of dyslipidaemias, for improving or preventing disturbances in the serum lipid profile.

The composition according to the invention will now be further exemplified without the examples given limiting the scope of the invention.

### Example 1:

The technical requirements necessary for the active ingredients in the composition T, according to the invention, to obtain therapeutic effects are:
- the red rice powder extract must contain min. 1% monacolin K;
- the sitosterol must contain min. 70% beta-sitosterol;
- taurine must be of quality standard USP 98.5 to 101%;
- niacin must be of quality standard USP30.

The composition, according to the invention, can be formulated into tablets containing:

| | |
|---|---|
| Red rice extract | 100 mg |
| Sitosterol | 100 mg |
| Niacin | 1.5 mg |
| Taurine | 150 mg |
| Cellulose | 81 mg |
| Silicon dioxide | 5.5 mg |
| Dicalcium phosphate | 81 mg |
| Hypromellose | 22 mg |

per tablet.

Tablets are prepared by wet granulation of the mixture of active ingredients with cellulose, silica and dicalcium phosphate. For the wet granulation an 8% aqueous hypromellose solution is used. The granules thus obtained are dried, mixed with the rest of the hypromellose and compressed.

### Example 2:

The composition T, according to the invention, can be introduced directly into hard-shelled capsules, using known techniques.

| | |
|---|---|
| Red rice extract | 100 mg |
| Sitosterol | 100 mg |
| Niacin | 1.5 mg |
| Taurine | 150 mg |

per capsule.

### Example 3:

For the formulation of the composition according to the invention, in soft capsules, the following composition will be used:

| | |
|---|---|
| Red rice extract | 100 mg |
| Sitosterol | 100 mg |
| Niacin | 1.5 mg |
| Taurine | 150 mg |
| Canola oil | 87 mg |
| Gelatine | 87 mg |
| Glycerin | 87 mg |
| Lecithin | 6.5 mg |
| Water | q. s. |

per soft capsule.

The mixture of active ingredients is dispersed using lecithin in the Canola oil and the resulting suspension is introduced into soft capsules made of gelatine, glycerol and water. The composition according to the invention in soft capsules has an extended release, which ensures effective plasma concentrations for a longer period of time between doses.

### Example 4:

The composition, according to the invention, can be formulated as drinkable suspension containing:

| | |
|---|---|
| Red rice extract | 100 mg |
| Sitosterol | 100 mg |
| Niacin | 1.5 mg |
| Taurine | 150 mg |
| Xanthan gum | 30 mg |
| Methylcellulose | 15 mg |
| Polysorbate | 15 mg |
| Lecithin | 15 mg |
| Sodium benzoate | 15 mg |
| Water | q. s. |

per one spoon of suspension.

The homogeneously mixed active ingredients are triturated with polysorbate and lecithin. The resulting mixture is incorporated into a aqueous mucilage made of xanthan gum and methylcellulose.

## Claims

1. Composition for the prevention and treatment of dyslipidaemias, **characterized in that** it is a combination of a compound that lowers the synthesis of endogenous cholesterol, a compound that reduces cholesterol absorption, a compound that increases the HDL and a compound that enhances cholesterol degradation, the ratio of the four compounds being 0.5...1: 0.5...1: 0.015: 1.5...2, expressed in parts by weight.

2. Composition, according to claim 1, **characterized in that** it consists of a combination of red rice extract which lowers cholesterol synthesis and significantly reduces serum lipids with a compound that reduces cholesterol absorption, a compound that contributes to the degradation of the cholesterol and a compound that increases the levels of HDL cholesterol and/or decreases the level of LDL cholesterol in a combination ratio of 1:1:0.15:1.5, expressed in parts by weight.

3. Composition according to claims 1-2, **characterized in that** the compound having a cholesterol-lowering absorption is chosen from sitosterol or sitostanol.

4. Composition according to claims 1-2, **characterized in that** the compound which contributes to the degradation of cholesterol is taurine.

5. Composition according to claims 1-2, **characterized in that** the compound that has the effect of increasing the HDL cholesterol is selected from niacin, carnitine or resveratrol.

6. Composition according to claims 1-5, **characterized in that** it consists of the combination of 28,45% red rice extract (or red rice yeast), which contains min. 1% monacolin K with 28.45% sitostanol containing minimum 70% beta-sitosterol, with 0.428% niacin and 42.67% taurine.

7. Composition according to claims 1-6, **characterized in that** it is formulated in oral dosage forms in combination with pharmaceutically acceptable excipients.

8. Use of the composition according to claims 1-7 for the preparation of oral dosage forms, as a food supplement or as a medicine for the treatment or prevention of dyslipidaemias, for improving or preventing disturbances in the serum lipid profile.
